# EUROPEAN PATENT SPECIFICATION

(11) **EP 3 514 209 B1**
(45) Date of publication and mention of the grant of the patent: **11.11.2020**
(21) Application number: 19159290.6
(22) Date of filing: 09.12.2016
(51) Int. Cl.: C09D 163/00, C08G 59/44, C08G 59/40, C07C 243/40, C07C 281/02, C07C 319/02, C07C 323/60, C07C 241/04

(54) **AMINIMIDE COMPOSITIONS**
AMINIMIDZUSAMMENSETZUNGEN
COMPOSITIONS AMINIMIDES

(30) Priority: 10.12.2015 US 201514964748; 10.12.2015 US 201514964795
(43) Date of publication of application: 24.07.2019
(62) Divisional of application: 16819786.1
(73) Proprietor: PPG Industries Ohio, Inc., Cleveland, OH 44111 (US)
(72) Inventor: CHAO, Tien-Chieh, Mars, Pennsylvania 16046 (US); NAKAJIMA, Masayuki, Wexford, Pennsylvania 15090 (US); ZHOU, Hongying, Allison Park, Pennsylvania 15101 (US); SWARUP, Shanti, Allison Park, Pennsylvania 15101 (US); DESAI, Umesh, Wailuku, Hawaii 96793 (US)
(74) Representative: f & e patent

(56) References cited:
- EP-A1- 2 199 313
- GB-A- 1 373 144
- JP-A- S58 142 955
- US-A- 3 485 806
- US-A- 3 628 992

## Description

### FIELD

The present invention relates to structural adhesive compositions, and more particularly to one-component compositions.

### BACKGROUND

Structural adhesives are utilized in a wide variety of applications to bond together two or more substrate materials. For example, structural adhesives may be used for binding together automotive or industrial components.

US 3,628,992 relates to the treatment of a filamentary substrate to improve the bonding properties thereof when utilized as the reinforcing element in the fabrication of vulcanized rubber structures.

GB 1 373 144 A relates to epoxy resin compositions of desirable hardening property and storage stability.

The present invention is directed towards adhesive compositions that provide sufficient bond strength and are easy to apply for use in bonding together substrate materials.

### SUMMARY

The scope of the protection is defined in the claims. The present invention is an adhesive composition comprising an epoxy compound and a monomeric compound comprising at least one aminimide functional group, wherein the monomeric compound reacts with the epoxy compound upon activation by an external energy source; wherein the monomeric compound is present in an amount from 2-8% by weight based on total weight of the adhesive composition, wherein the adhesive composition further comprises a reaction product of reactants comprising an amidine and a second component.

The present invention also is an adhesive composition comprising an epoxy compound; an aminimide-containing compound present in an amount of from 2% to 8% by weight based on total weight of the adhesive composition; and a reaction product of reactants comprising an amidine and a second component, wherein the epoxy, the aminimide-containing compound, and the reaction product react upon activation by an external energy source.

Also disclosed are adhesives formed from the adhesive compositions and methods of forming a bonded substrate using the adhesive compositions disclosed herein.

### DETAILED DESCRIPTION

For purposes of the following detailed description, it is to be understood that the invention may assume various alternative variations and step sequences except where expressly specified to the contrary. Moreover, other than in any operating examples, or where otherwise indicated, all numbers expressing, for example, quantities of ingredients used in the specification and claims, are to be understood as being modified in all instances by the term "about". Accordingly, unless indicated to the contrary, the numerical parameters set forth in the following specification and attached claims are approximations that may vary depending upon the desired properties to be obtained by the present invention. At the very least, and not as an attempt to limit the application of the doctrine of equivalents to the scope of the claims, each numerical parameter should at least be construed in light of the number of reported significant digits and by applying ordinary rounding techniques. Where a closed or open-ended numerical range is described herein, all numbers, values, amounts, percentages, subranges and fractions within or encompassed by the numerical range are to be considered as being specifically included in and belonging to the original disclosure of this application as if these numbers, values, amounts, percentages, subranges and fractions had been explicitly written out in their entirety.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard variation found in their respective testing measurements.

It is also understood that, as used herein, a plural term can encompass its singular counterpart and vice versa. For example, although reference is made herein to "a" trivalent nitrogen, "an" anhydride functional material, and "a" cyclic ester, a combination (a plurality) of these components can be used in the present invention.

In addition, in this application, the use of "or" means "and/or" unless specifically stated otherwise, even though "and/or" may be explicitly used in certain instances.

As used herein, "including," "containing" and like terms are understood in the context of this application to be synonymous with "comprising" and are therefore open-ended and do not exclude the presence of additional undescribed or unrecited elements, materials, ingredients or method steps. As used herein, "consisting of' is understood in the context of this application to exclude the presence of any unspecified element, ingredient or method step. As used herein, "consisting essentially of' is understood in the context of this application to include the specified elements, materials, ingredients or method steps "and those that do not materially affect the basic and novel characteristic(s)" of what is being described.

As used herein, unless indicated otherwise, the term "substantially free," when used with respect to the synthesis of an aminimide by any of reactions I-VII of the present invention or production of one of the aminimides represented by formulae V-XI of the present invention, means that a particular material is not purposefully added and is present in a trace amount of 5% or less based on total composition weight.

As used herein, unless indicated otherwise, the term "substantially free," when used with respect to the adhesive composition of the present invention, means that a particular material is not purposefully added to a composition, and is only present as an impurity in a trace amount of less than 1% by weight based on a total weight of the composition.

As used herein, unless indicated otherwise, the term "completely free" means that a composition does not comprise a particular material, i.e., the composition comprises 0% of such material based on total composition weight.

As used herein, the terms "on," "onto," "applied on," "applied onto," "formed on," "deposited on," "deposited onto," mean formed, overlaid, deposited, or provided on but not necessarily in contact with the surface. For example, an adhesive composition "applied onto" a substrate does not preclude the presence of one or more other intervening coating layers of the same or different composition located between the adhesive composition and the substrate.

As used herein, the term "structural adhesive" means an adhesive producing a load-bearing joint having a lap shear strength of greater than 5 MPa, as determined by using an Instron 5567 machine in tensile mode with a pull rate of 10 mm per minute.

As used herein, "alkyl" refers to an optionally substituted hydrocarbon chain that may be linear or branched.

As used herein, "aromatic" refers to a hydrocarbon having a delocalized conjugated π-system with alternating double and single bonds between carbon atoms forming one or more coplanar hydrocarbon rings.

As used herein, "cycloaliphatic" refers to a hydrocarbon that comprises one or more hydrocarbon rings that are not aromatic.

As defined herein, a "1K" or "one-component" adhesive composition, is a composition in which all of the ingredients may be premixed and stored and wherein the reactive components do not readily react at ambient or slightly thermal conditions, but instead only react upon activation by an external energy source. In the absence of activation from the external energy source, the composition will remain largely unreacted (having less than a 100 % increase in viscosity when stored at 25°C for 90 days, where viscosity is measured with an Anton Paar Physica MCR 301 rheometer with 25mm parallel plate and 1 mm gap at the following shear conditions: Conditioning: Rotation with shear rate at 0.1 s⁻¹ for 60 seconds; Tempering: No shear for 240 seconds; Amplitude test: Oscillation with log increasing strain γ from 0.01 to 10% in 90 seconds (data measured every 3 seconds); Shear phase: Oscillation with 10% strain (γ) at 10 Hz for 120 seconds (data measured every 10 seconds); Re-conditioning: Rotation with shear rate at 0.1 s⁻¹ for 60 seconds; Regenerated mode: Oscillation with 0.05% strain (γ) for 120 seconds (data measured every 10 seconds)). External energy sources that may be used to promote the curing reaction include, for example, radiation (i.e., actinic radiation such as ultraviolet light) and/or heat. As further defined herein, ambient conditions generally refer to room temperature and humidity conditions or temperature and humidity conditions that are typically found in the area in which the adhesive is being applied to a substrate, e.g., at 20°C to 40°C and 20% to 80% relative humidity, while slightly thermal conditions are temperatures that are slightly above ambient temperature but are generally below the curing temperature for the adhesive composition (i.e. in other words, at temperatures and humidity conditions below which the reactive components will readily react and cure, e.g., > 40°C and less than 100°C at 20% to 80% relative humidity).

As used herein "monomer" refers generally to a component that can be polymerized with another polymerizable component such as another monomer or a polymer to form a compound that comprises residues of the monomeric or polymeric components, respectively.

As used herein "polymer" refers generally to prepolymers, oligomers, homopolymers, copolymers, or combinations thereof; the prefix "poly" refers to two or more repeating units derived from one or more monomers in the molecule.

As used herein, "(meth)acrylate" includes both acrylate and methacrylate monomers.

As used herein, the term "heteroatom" refers to an oxygen atom, a nitrogen atom, or a sulfur atom.

As used herein, the term "diluent" refers to a material that substantially dissolves the reactants and/or polymers formed therefrom but that is inert with the reactants and/or polymers formed therefrom. The term "substantially dissolve," when used with respect to the diluent, means that the material, *i.e.* a particular reactant or polymer, becomes incorporated into the diluent so as to form a solution. This means that, according to the present invention, at least 80% of the material may be dissolved in the diluent, or in some cases, at least 90% of the material may be dissolved in the diluent, or in some cases, at least 95% of the material may be dissolved in the diluent based on the total weight of the material in the mixture.

As used herein, the term "aminimide" refers to a molecule, *i.e.* a monomer or a polymer, that comprises at least one aminimide functional group. As used herein, an "aminimide functional group" comprises an anionic nitrogen bonded to a cationic nitrogen and a carbonyl group according to formula I: 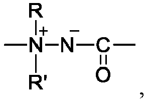 wherein R and R' may be the same or different and may be an alkyl group or an aryl group. For clarity, an aminimide may comprise additional functional groups in addition to the aminimide functional group(s).

As used herein, the term "carbonyl group" refers to a functional group comprising a carbon atom double-bonded to an oxygen atom, which is common to several classes of compounds including, but not being limited to as aldehydes, ketones, carboxylic acids, acid anhydrides, esters, *etc.*

As used herein, "monofunctional," when used with respect to the number of aminimide functional groups a particular monomer or polymer comprises, means a monomer or polymer comprising more than zero (0) aminimide functional groups but fewer than two (2) aminimide functional groups, such as, for example, one aminimide functional group per molecule.

As used herein, "bifunctional," when used with respect to the number of aminimide functional groups a particular monomer or polymer comprises, means a monomer or polymer comprising two (2) aminimide functional groups per molecule.

As used herein, "polyfunctional," when used with respect to the number of aminimide functional groups a particular monomer or polymer comprises, means a monomer or polymer comprising more than two (2) aminimide functional groups per molecule.

As used herein, "aminimide" containing compounds refer to compounds that act as catalysts for epoxy resins.

As used herein, the term "catalyst" means a substance that increases the rate of a chemical reaction without itself undergoing any permanent chemical change.

As used herein, the term "trivalent nitrogen" refers to a nitrogen atom bound to one further nitrogen atom and two carbon atoms.

As used herein, the term "molecular weight" means the theoretical number average molecular weight (Mₙ) as determined by Gel Permeation Chromatography using Waters 2695 separation module with a Waters 410 differential refractometer (RI detector), polystyrene standards having molecular weights of from approximately 800 g/mol to 900,000 g/mol, tetrahydrofuran (THF) as the eluent at a flow rate of 1 mL/min, and two PG Gel Mixed C columns for separation.

As used herein, the term "volatile" refers to an organic substance having an initial boiling point of less than or equal to 250 °C at a standard atmospheric pressure of 101.3 kPa, whereas the term "non-volatile" refers to any organic substance having an initial boiling point of above 250 °C at a standard atmospheric pressure of 101.3 kPa.

As stated above, disclosed herein is an adhesive composition. The adhesive composition of the present invention comprises an epoxy compound and a monomeric compound comprising at least one aminimide functional group, wherein the monomeric compound comprising the at least one aminimide functional group reacts with the epoxy compound upon activation by an external energy source, and wherein the compound is present in an amount from 2-8% by weight based on total weight of the adhesive composition, and wherein the adhesive composition further comprises a reaction product of reactants comprising an amidine and a second component.

The adhesive composition comprises an epoxy compound. Suitable epoxy compounds that may be used include monoepoxides, polyepoxides, or combinations thereof.

Suitable monoepoxides that may be used to form the adhesive composition of the present invention include monoglycidyl ethers of alcohols and phenols, such as phenyl glycidyl ether, n-butyl glycidyl ether, cresyl glycidyl ether, isopropyl glycidyl ether, glycidyl versatate, for example, CARDURA E available from Shell Chemical Co., and glycidyl esters of monocarboxylic acids such as glycidyl neodecanoate, and mixtures of any of the foregoing.

Suitable polyepoxides that may be used to form the adhesive composition of the present invention include polyglycidyl ethers of Bisphenol A, such as Epon® 828 and 1001 epoxy resins, and Bisphenol F diepoxides, such as Epon® 862, which are commercially available from Hexion Specialty Chemicals, Inc. Other useful polyepoxides include polyglycidyl ethers of polyhydric alcohols, polyglycidyl esters of polycarboxylic acids, polyepoxides that are derived from the epoxidation of an olefinically unsaturated alicyclic compound, polyepoxides containing oxyalkylene groups in the epoxy molecule, and combinations thereof.

In addition to the polyepoxides described above, additional polymers containing pendant epoxy groups also may be used to form the adhesive composition of the present invention. These polymers may be made by copolymerizing a variety of polymerizable ethylenically unsaturated monomers at least one of which is an epoxy containing monomer, e.g., glycidyl (meth)acrylate or allyl glycidyl ether. An example of such an additional polymer includes but is not limited to Epon® 1007.

Other useful epoxides that may be used to form the adhesive composition of the present invention include polyepoxides and are disclosed, for example, in U.S. Publication No. US/2014/0150970 at paragraphs [0023] to [0027].

Useful polyols that may be used to form an epoxy-functional resin for use in the adhesive composition include diols, tetraols and higher functional polyols. The polyols can be based on a polyether chain derived from ethylene glycol, propylene glycol, butylenes glycol, hexylene glycol and the like and mixtures thereof. The polyol can also be based on a polyester chain derived from ring opening polymerization of caprolactone. Suitable polyols may also include polyether polyol, polyurethane polyol, polyurea polyol, acrylic polyol, polyester polyol, polybutadiene polyol, hydrogenated polybutadiene polyol, polycarbonate polyols, polysiloxane polyol, and combinations thereof. Polyamines corresponding to polyols can also be used, and in this case, amides instead of carboxylic esters will be formed with acids and anhydrides.

Suitable diols that may be utilized to form the epoxy-functional resin for use in the adhesive composition are diols having a hydroxyl equivalent weight of between 30 and 1000. Exemplary diols having a hydroxyl equivalent weight from 30 to 1000 include diols sold under the trade name Terathane ®, including Terathane® 250, available from Invista. Other exemplary diols having a hydroxyl equivalent weight from 30 to 1000 include ethylene glycol and its polyether diols, propylene glycol and its polyether diols, butylenes glycol and its polyether diols, hexylene glycols and its polyether diols, polyester diols synthesized by ring opening polymerization of caprolactone, and urethane diols synthesized by reaction of cyclic carbonates with diamines. Combination of these diols and polyether diols derived from combination various diols described above could also be used. Dimer diols may also be used including those sold under trade names Pripol® and Solvermol™ available from Cognis Corporation.

Polytetrahydrofuran-based polyols sold under the trade name Terathane®, including Terathane® 650, available from Invista, may be used to form the adhesive composition of the present invention. In addition, polyols based on dimer diols sold under the trade names Pripol® and Empol®, available from Cognis Corporation, or bio-based polyols, such as the tetrafunctional polyol Agrol 4.0, available from BioBased Technologies, may also be utilized.

Useful anhydride compounds to functionalize the polyol with acid groups include hexahydrophthalic anhydride and its derivatives (e.g. methyl hexahydrophthalic anhydride); phthalic anhydride and its derivatives (e.g. methyl phthalic anhydride); maleic anhydride; succinic anhydride; trimelletic anhydride; pyromelletic dianhydride (PMDA); 3,3', 4,4'-oxydiphthalic dianhydride (ODPA); 3,3',4,4'-benzopherone tetracarboxylic dianhydride (BTDA); and 4,4'-diphthalic(hexamfluoroisopropylidene)anhydride (6FDA). Useful diacid compounds to functionalize the polyol with acid groups include phthalic acid and its derivatives (e.g. methyl phthalic acid), hexahydrophthalic acid and its derivatives (e.g. methyl hexahydrophthalic acid), maleic acid, succinic acid, adipic acid, etc. Any diacid and anhydride can be used.

According to the present invention, the epoxy compound may be present in the adhesive composition in an amount of at least 50% by weight based on total composition weight, such as at least 60% by weight, such as at least 70% by weight, and in some cases may be no more than 95% by weight based on total composition weight, such as no more than 90% by weight, such as no more than 85% by weight. According to the present invention, the epoxy compound may be present in the adhesive composition in an amount from 50% to 95% by weight based on the total composition weight, such as from 60% to 90%, such as from 70% to 87%.

The molecular weight of the epoxy compound used to form the adhesive composition of the present invention may be at least 44, such as at least 58, and in some cases may be no more than 5000, such as no more than 3000, such as no more than 1000. According to the present invention, the molecular weight of the epoxy compound may be from 44 to 5000, such as from 58 to 3000, such as from 58 to 1000.

The epoxy compound used to form the adhesive composition of the present invention may have an epoxy equivalent weight (EEW) of at least 44, such as at least 58, and in some cases may be no more than 2500, such as no more than 1500, such as no more than 500. According to the present invention, the epoxy compound may have an EEW of from 44 to 2500, such as from 58 to 1500, such as from 58 to 500. As used herein, EEW refers to the molecular weight of the epoxide compound divided by the number of epoxy groups per molecule.

The adhesive composition also comprises a compound containing at least one aminimide functional group, i.e., the compound may be a monofunctional aminimide, a difunctional aminimide, or a polyfunctional aminimide. Suitable compounds containing at least one aminimide functional group include monomeric compounds. Useful monomeric aminimide-containing compounds may comprise a reaction product of reactants comprising a monofunctional epoxy and hydrazine with ester functionality. Optionally, the aminimide-containing compound of the present invention may comprise at least one functional group in addition to the aminimide functional group(s), such as an acid functional group, an hydroxyl functional group, an amine functional group, a mercaptofunctional group, or combinations thereof.

According to the present invention, the aminimide-containing compound is present in the adhesive composition in an amount of at least 2% by weight based on total weight of the adhesive composition, such as at least 2.5% by weight, such as at least 3% by weight, and is present in an amount of no more than 8% by weight based on total weight of the adhesive composition, such as no more than 7.5%, such as no more than 7% by weight. According to the present invention, the aminimide-containing compound is present in the adhesive composition in an amount of from 2% to 8% by weight based on total weight of the adhesive composition, such as from 2.5% to 7.5%, such as from 3% to 7%.

According to the present invention, the aminimide-containing compound may chemically react with the epoxy compound upon activation by an external energy source, such as for example, radiation and/or heat. Optionally, for example, the aminimide-containing compound may chemically react with the epoxy compound upon exposure to a temperature of at least 100°C, such as at least 110 °C, such as at least 120 °C, such as at least 130 °C, and in some cases may be exposed to a temperature of no more than 200 °C, such as no more than 190 °C, such as no more than 180 °C, such as no more than 170 °C. According to the present invention, the aminimide-containing compound may chemically react with the epoxy compound upon exposure to a temperature of from 100 °C to 200 °C, such as from 110 °C to 190 °C, such as from 120 °C to 180 °C, such as from 130 °C to 170 °C.

In the present invention, the adhesive composition further comprises a reaction product of reactants comprising an amidine and a second component. Useful amidines include, but are not limited to 1,8-diazabicyclo[5.4.0]undec-7-ene; 1,5-diazabicyclo[4.3.0]non-5-ene; 1,5,7-triazabicyclo[4.4.0]dec-5-ene; or combinations thereof. Useful second components include but are not limited to a phenol-containing compound such as for example a phenol formaldehyde resin such as Novolac resins, carbonic acid, a salt of carbonic acid, carbonate, or combinations thereof. Optionally, in the present invention, the amidine and the second component form an amidine salt. According to the present invention, the amidine-containing compound may be present in the adhesive composition in an amount of at least 1% by weight based on total weight of the adhesive composition, such as at least 1.25% by weight, such as at least 1.5% by weight, and in some cases may be present in an amount of no more than 4% by weight based on total weight of the adhesive composition, such as no more than 3.75%, such as no more than 3.5% by weight. According to the present invention, the amidine-containing compound may be present in the adhesive composition in an amount of from 1% to 4% by weight based on total weight of the adhesive composition, such as from 1.25% to 3.75%, such as from 1.5% to 3.5%.

Optionally, the adhesive composition also may comprise rubber particles having a core-shell structure. Suitable core-shell rubber particles may be comprised of butadiene rubber or other synthetic rubbers, such as styrene-butadiene and acrylonitrile-butadiene and the like. The type of synthetic rubber and the rubber concentration is not limited as long as the particle size falls within the specified range as illustrated below.

According to the present invention, the average particle size of the rubber particles may be from 0.02 to 500 microns (20 nm to 500,000 nm), for example, the reported particle size for rubber particles provided by Kanekea Texas Corporation, as measured by standard techniques known in the industry, such as, for example, according to ISO 13320 and ISO 22412.

According to the present invention, the core-shell rubber particles may optionally be included in an epoxy carrier resin for introduction into the adhesive composition. Suitable finely dispersed core-shell rubber particles in an average particle size ranging from 50 nm to 250 nm may be master-batched in epoxy resin such as aromatic epoxides, phenolic novolac epoxy resin, bisphenol A and/or bisphenol F diepoxide, and/or aliphatic epoxides, which include cyclo-aliphatic epoxides, at concentrations ranging from 5% to 40% rubber particles by weight based on the total weight of the rubber dispersion, such as from 20% to 35%. Suitable epoxy resins may also include a mixture of epoxy resins. When utilized, the epoxy carrier resin may be an epoxy-containing component of the present invention such that the weight of the epoxy-containing component present in the structural adhesive composition includes the weight of the epoxy carrier resin.

Exemplary non-limiting commercial core-shell rubber particle products using poly(butadiene) rubber particles that may be utilized in the adhesive composition include a core-shell poly(butadiene) rubber dispersion (25% rubber by weight) in bisphenol F (commercially available as Kane Ace MX 136), a core-shell poly(butadiene) rubber dispersion (33% rubber by weight) in Epon® 828 (commercially available as Kane Ace MX 153), a core-shell poly(butadiene) rubber dispersion (37% rubber by weight) in bisphenol A (commercially available as Kane Ace MX 257), and a core-shell poly(butadiene) rubber dispersion (37% rubber by weight) in bisphenol F (commercially available as Kane Ace MX 267), each available from Kaneka Texas Corporation.

Exemplary non-limiting commercial core-shell rubber particle products using styrene-butadiene rubber particles that may be utilized in the adhesive composition include a core-shell styrene-butadiene rubber dispersion (33% rubber by weight) in low viscosity bisphenol A (commercially available as Kane Ace MX 113), a core-shell styrene-butadiene rubber dispersion (25% rubber by weight) in bisphenol A (commercially available as Kane Ace MX 125), a core-shell styrene-butadiene rubber dispersion (25% rubber by weight) in D.E.N.™-438 phenolic novolac epoxy (commercially available as Kane Ace MX 215), a core-shell styrene-butadiene rubber dispersion (25% rubber by weight) in Araldite® MY-721 multi-functional epoxy (commercially available as Kane Ace MX 416), a core-shell styrene-butadiene rubber dispersion (25% rubber by weight) in MY-0510 multi-functional epoxy (commercially available as Kane Ace MX 451), a core-shell styrene-butadiene rubber dispersion (25% rubber by weight) in Syna Epoxy 21 Cyclo-aliphatic Epoxy from Synasia (commercially available as Kane Ace MX 551), and a core-shell styrene-butadiene rubber dispersion (25% rubber by weight) in polypropylene glycol (MW 400) (commercially available as Kane Ace MX 715), each available from Kaneka Texas Corporation.

According to the present invention, if rubber particles having a core-shell structure are included in the adhesive composition, the rubber particles may be present in the adhesive composition in an amount of at least 10% by weight based on total composition weight, such as at least 20% by weight, such as at least 25% by weight, and in some cases may be present in the adhesive composition in an amount of no more than 45% by weight based on total composition weight, such as no more than 40% by weight, such as no more than 35% by weight. According to the present invention, rubber particles having a core-shell structure may be present in the adhesive composition, if at all, in an amount of from 10% to 45% by weight based on the total composition weight, such as from 20% to 40% by weight, such as from 25% to 35% by weight.

Optionally, the adhesive formulation may also include epoxy compounds or resins that are not incorporated into or reacted as a part of any of the components described above, including epoxy-functional polymers that can be saturated or unsaturated, cyclic or acyclic, aliphatic, alicyclic, aromatic or heterocyclic. The epoxy-functional polymers can have pendant or terminal hydroxyl groups, if desired. They can contain substituents such as halogen, hydroxyl, and ether groups. A useful class of these materials includes polyepoxides comprising epoxy polyethers obtained by reacting an epihalohydrin (such as epichlorohydrin or epibromohydrin) with a di- or polyhydric alcohol in the presence of an alkali. Suitable polyhydric alcohols include polyphenols such as resorcinol; catechol; hydroquinone; bis(4-hydroxyphenyl)-2,2-propane, i.e., Bisphenol A; bis(4-hydroxyphenyl)-1,1-isobutane; 4,4-dihydroxybenzophenone; bis(4-hydroxyphenol)-1,1-ethane; bis(2-hydroxyphenyl)-methane and 1,5-hydroxynaphthalene.

Frequently used polyepoxides include polyglycidyl ethers of Bisphenol A, such as Epon® 828 epoxy resin which is commercially available from Hexion Specialty Chemicals, Inc. and having a number average molecular weight of about 400 and an epoxy equivalent weight of about 185-192. Other useful polyepoxides include polyglycidyl ethers of other polyhydric alcohols, polyglycidyl esters of polycarboxylic acids, polyepoxides that are derived from the epoxidation of an olefinically unsaturated alicyclic compound, polyepoxides containing oxyalkylene groups in the epoxy molecule, epoxy novolac resins, and polyepoxides that are partially defunctionalized by carboxylic acids, alcohol, water, phenols, mercaptans or other active hydrogen-containing compounds to give hydroxyl-containing polymers.

According to the present invention, reinforcement fillers may be added to the adhesive composition. Useful reinforcement fillers that may be introduced to the adhesive composition to provide improved mechanical properties include fibrous materials such as fiberglass, fibrous titanium dioxide, whisker type calcium carbonate (aragonite), and carbon fiber (which includes graphite and carbon nanotubes). In addition, fiber glass ground to 5 microns or wider and to 50 microns or longer may also provide additional tensile strength. Such reinforcement fillers, if utilized, may be present in the adhesive composition in an amount of at least 0.1% by weight based on total composition weight, such as at least 0.5% by weight, such as at least 1% by weight and, in some cases, may be present in an amount of no more than 5% by weight based on total composition weight, such as no more than 4.5% by weight, such as no more than 4% by weight. According to the present invention, reinforcement fillers may be present in the adhesive composition in an amount of from 0.1% by weight to 5% by weight based on total composition weight, such as from 0.5% by weight to 4.5% by weight, such as from 1% by weight to 4% by weight.

Optionally, according to the present invention, additional fillers, thixotropes, colorants, tints and/or other materials also may be added to the adhesive composition.

Useful thixotropes that may be used include untreated fumed silica and treated fumed silica, Castor wax, clay, and organo clay. In addition, fibers such as synthetic fibers like aromatic polyamide fibers (such as those commercially available as Aramid® fiber and Kevlar® fiber), acrylic fibers, and engineered cellulose fiber may also be utilized.

Useful colorants or tints may include red iron pigment, titanium dioxide, calcium carbonate, phthalocyanine green and phthalocyanine blue.

Useful fillers that may be used in conjunction with thixotropes may include inorganic fillers such as inorganic clay or silica.

Exemplary other materials that may be utilized include, for example, calcium oxide and carbon black.

The adhesive composition of the present invention may comprise, or in some cases may consist essentially of, or in some cases may consist of, an adhesive composition comprising an epoxy compound, an aminimide-containing monomeric compound present in an amount of from 2% to 8% by weight based on total weight of the adhesive composition, and a reaction product of reactants comprising an amidine and a second component, wherein the epoxy, the aminimide-containing monomeric compound, and the reaction product react upon activation by an external energy source.

The present invention may also be a method for preparing an adhesive composition comprising, or in some cases consisting of, or in some cases consisting essentially of, mixing an epoxy and a compound comprising at least one aminimide functional group. According to the present invention, the epoxy compound may be any of the monoepoxides or polyepoxides described above. According to the present invention, the compound comprising the at least one aminimide functional group is monomeric, as described above, and has at least one aminimide functional groups, as described above.

As stated above, the present disclosure is directed to one-component structural adhesive compositions that are used to bond together two substrate materials for a wide variety of potential applications in which the bond between the substrate materials provides particular mechanical properties related to elongation, tensile strength, lap shear strength, T-peel strength, modulus, or impact peel strength. The structural adhesive may be applied to either one or both of the substrate materials being bonded such as, by way of non-limiting example, components of an automobile frame. The pieces are aligned and pressure and spacers may be added to control bond thickness. The adhesive may be cured using an external source such as an oven (or other thermal means) or through the use of actinic radiation (UV light, etc.). Suitable substrate materials that may be bonded by the structural adhesive compositions of the present invention include, but are not limited to, materials such as metals or metal alloys, natural materials such as wood, polymeric materials such as hard plastics, or composite materials wherein each of the first and the second substrate material may be independently selected from these materials. The structural adhesives of the present invention are particularly suitable for use in various automotive or industrial applications.

The present invention may also be a method for forming a bonded substrate comprising, or in some cases consisting of, or in some cases consisting essentially of, applying the adhesive composition described above to a first substrate; contacting a second substrate to the adhesive composition such that the adhesive composition is located between the first substrate and the second substrate; and curing the adhesive composition.

The adhesive composition described above may be applied alone or as part of an adhesive system that can be deposited in a number of different ways onto a number of different substrates. The adhesive system may comprise a number of the same or different adhesive layers. An adhesive layer is typically formed when an adhesive composition that is deposited onto the substrate is at least partially cured by methods known to those of ordinary skill in the art (e.g., by exposure to thermal heating).

The adhesive composition can be applied to the surface of a substrate in any number of different ways, non-limiting examples of which include brushes, rollers, films, pellets, spray guns and applicator guns.

After application to the substrate, the adhesive composition can be at least partially cured, such as by baking and/or curing at elevated temperature for any desired time period sufficient to at least partially cure the adhesive composition on the substrate (e.g., from 5 minutes to 1 hour), such as at a temperature of at least 100°C, such as at least 110 °C, such as at least 120 °C, such as at least 130 °C, and in some cases may be exposed to a temperature of no more than 200 °C, such as no more than 190 °C, such as no more than 180 °C, such as no more than 170 °C. According to the present invention, the adhesive composition can be at least partially cured, such as by baking and/or curing at elevated temperature for any desired time period sufficient to at least partially cure the adhesive composition on the substrate (e.g., from 5 minutes to 1 hour), such as at a temperature of from 100 °C to 200 °C, such as from 110 °C to 190 °C, such as from 120 °C to 180 °C, such as from 130 °C to 170 °C.

It was surprisingly discovered that the adhesive composition of the present invention resulted in improved stability of the composition, as demonstrated by improved complex viscosity, as well as improved mechanical properties of the cured adhesive, such as improved lap shear.

The adhesive composition of the present invention (as measured with an Anton Paar Physica MCR 301 rheometer with 25 mm parallel plate and 1 mm gap) may have a change in complex viscosity (η^{∗} A1, measured at the condition of y = 0.05% (at 21 seconds) after 3 days at 43°C (conditioned at 35°C before each measurement)) of no more than 5x the initial value, such as no more than 4x the initial value, such as no more than 3x the initial value, such as no more than 2x the initial value.

According to the present invention, after the adhesive composition is applied to a substrate and at least partially cured, the bonded substrate(s) may demonstrate a lap shear of at least 7 as measured according to ISO 4587 test method, such as at least 12, such as at least 14, such as at least 16, such as at least 22.

Whereas particular embodiments have been described above for purposes of illustration, it will be evident to those skilled in the art that numerous variations of the details of the coating composition, coating, and methods disclosed herein may be made without departing from the scope in the appended claims.

Illustrating the invention are the following examples that are not to be considered as limiting the invention to their details. All parts and percentages in the examples, as well as throughout the specification, are by weight unless otherwise indicated.

### EXAMPLES

The following Examples G, H, I, O and P provide descriptions of the synthesis of aminimide-containing materials and 1,8-diazabicyclo[5.4.0]undec-7-ene (DBU) salts which were used to prepare the adhesive compositions of Examples 2-4 and 10 as well as Comparative Example 11, as described in further detail herein. In each Example, the final powder was ground with a mortar and pestle, and was shaken on a sieve shaker (Retsch AS 300) at an amplitude of 1 mm/g and a sieve of <125 µm. "1 foot" is equivalent to "0.3048 m".

### Synthesis Examples G, H, I, O and P

### Example G

Compound A was synthesized as follows: Into a 500-milliliter, 4-necked flask equipped with a stirrer, a condenser, a nitrogen inlet, and a thermocouple in a heating mantle, was charged 68.0 grams of methyl benzoate, 50.06 grams of 1,1-dimethylhydrazine (62% by weight solution available from Lonza Group Ltd.), 31.6 grams of propylene oxide, and 310 grams of isopropanol. Agitation and a nitrogen flow of 0.2 scft / min ("scft" means standard cubic feet) were started. The mixture was stirred at 20 °C for 2 hours and then at 55 °C for 24 hours. The reaction progress was monitored by a Gas Chromatographer. After completion of the reaction, the reaction mixture was concentrated by evaporation of solvent, and the residual white solid was recrystallized from ethyl acetate. A white powder was obtained in a yield of 56% by weight. The solid was further ground into fine powder having a sieve fraction of < 125 µm, as described above.

### Example H

Into a 500-milliliter, 4-necked flask equipped with a stirrer, a condenser, a nitrogen inlet, and a thermocouple in a heating mantle, was charged 54.4 grams of methyl benzoate, 40.6 grams of 1-aminopiperidine, 23.21 grams of propylene oxide, and 250 grams of isopropanol. Agitation and a nitrogen flow of 0.2 scft / min ("scft" means standard cubic feet) were started. The mixture was stirred at 20 °C for 2 hours and then at 55 °C for 24 hours. The reaction progress was monitored by a Gas Chromatographer. After completion of the reaction, the reaction mixture was concentrated by evaporation of solvent, and the residual white solid was recrystallized from ethyl acetate. A white powder was obtained in a yield of 59% by weight. The solid was further ground into fine powder having a sieve fraction of < 125 µm, as described above.

### Example I

Into a 500-milliliter, 4-necked flask equipped with a stirrer, a condenser, a nitrogen inlet, and a thermocouple in a heating mantle, was charged 54.4 grams of dimethyl terephthalate, 54.31 grams of 1,1-dimethylhydrazine (62% solution), 65.09 grams of propylene oxide, and 203 grams of isopropanol. Agitation and a nitrogen flow of 0.2 scft / min ("scft" means standard cubic feet) were started. The mixture was stirred at 20 °C for 2 hours and then at 80 °C for 8 hours. The reaction progress was monitored by a Gas Chromatographer. After completion of the reaction, the reaction mixture was concentrated by evaporation of solvent, and the residual white solid was recrystallized from ethyl acetate. A white powder was obtained in a yield of 48% by weight. The solid was further ground into fine powder having a sieve fraction of < 125 µm, as described above.

### Example O

Into a 500-milliliter, 4-necked kettle equipped with a stirrer, a condenser, a nitrogen inlet, and a thermocouple in a heating mantle, was charged 165.0 grams of FRJ-425 (a phenol formaldehyde novolac resin commercially available from SI Group) and heated to 160 °C to become a liquid. 117.6 grams of DBU (Industrial grade from BASF) were added into reaction mixture drop wise. After addition, the reaction mixture was heated to 180 °C and held for 30 minutes. After holding, the liquid was poured out onto aluminum foil and formed a solid at room temperature. The solid was ground into fine powder having a sieve fraction of < 125 µm, as described above.

### Example P

Into a 2000-milliliter, 4-necked kettle equipped with a stirrer, a condenser, a nitrogen inlet, and a thermocouple, was charged 50.6 grams of DBU (Industrial grade from BASF), 6.66 grams of DI water, and 300 grams of ethyl acetate. 50 grams of dry ice were partially added into reaction mixture and white precipitate formed. After addition, the reaction mixture was held at room temperature for 1 hour. After holding, the white solid was collected by filtration. The solid was washed with ethyl acetate twice (2X30 mL) and dried in vacuum oven. A white powder was obtained in a yield of 97% by weight. The solid was ground into fine powder having a sieve fraction of < 125 µm, as described above.

The following examples show the preparation of various adhesive compositions prepared with aminimides of the invention as catalysts for epoxy resins. Components were added in the order shown in Table 1.

### Adhesive Examples 1, 2-4, 10 and 11

Six (6) adhesive compositions were prepared from the mixture of ingredients shown in Table 1.

**Table 1: Adhesive compositions**

| Components | Comp. Ex. 1 | Ex. 2 | Ex. 3 | Ex. 4 | Ex. 10 | Comp. Ex. 11 |
|---|---|---|---|---|---|---|
| Kane Ace MX-153¹ | 48 | 48 | 48 | 48 | 48 | 48 |
| Epoxy resin² | 18.4 | 18.4 | 18.4 | 18.4 | 18.4 | 18.4 |
| TINT-AYD ST 8703³ | 0.1 | 0.1 | 0.1 | 0.1 | 0.1 | 0.1 |
| Dyhard SF 100⁴ | 3.4 | 3.4 | 3.4 | 3.4 | 3.4 | 3.4 |
| Diuron⁵ | 0.3 | 0.3 | 0.3 | 0.3 | 0.3 | 0.3 |
| Synthesis Example G | - | 2 | - | - | 2 | 2 |
| Synthesis Example H | - | - | 2 | - | - | - |
| Synthesis Example I | - | - | - | 2 | - | - |
| Synthesis Example J | - | - | - | - | - | - |
| Synthesis Example K | - | - | - | - | - | - |
| Synthesis Example L | - | - | - | - | - | - |
| Synthesis Example M | - | - | - | - | - | - |
| Synthesis Example N | - | - | - | - | - | - |
| Synthesis Example O | - | 2 | 2 | 2 | - | - |
| Synthesis Example P | - | - | - | - | 2 | - |
| Mica⁶ | 1 | 1 | 1 | 1 | 1 | 1 |
| Calcium Oxide⁷ | 2 | 2 | 2 | 2 | 2 | 2 |
| TOTAL | 73.2 | 77.2 | 77.2 | 77.2 | 77.2 | 75.2 |

| | | | | | | |
|---|---|---|---|---|---|---|
| 1: Blend of bisphenol A based epoxy resin and core-shell rubber available from Kaneka Corporation 2: Epoxy capped polyester 3: Phthalo green dye available from Elementis Specialties 4: Cyanoguanidine available from Alz Chem 5: Dimethyl-1,1,3-(3,4-dichlorophenyl) available from Alz Chem 6: Potassium alumina silica available from Pacer Corporation 7: Available from Mississippi Lime Company | | | | | | |

Adhesive lap shear specimens were prepared by applying adhesive on 20 mm x 90 mm x 0.8 mm size of hot dip galvanized (HDG) steel panels. The adhesive was applied to one end of a panel covering the whole width and 10 mm from the end. Glass beads with an average diameter of 0.25 mm were lightly sprinkled onto the adhesive to help maintain thickness. Another panel without adhesive was then placed over the adhesive area in an end-to-end fashion that would result in a 10 mm x 20 mm bond area. The joints were secured with metal clips with excess adhesive cleaned. They were then placed in an oven and baked according to specifications. The baked adhesive specimens were tested in an Instron 5567 machine in tensile mode with a pull rate of 10 mm per minute.

The adhesive lap shear strengths for the above compositions are shown in Table 2.

**Table 2: Lap Shear**

| | Lap shear Tensile (MPa) | | | | | |
|---|---|---|---|---|---|---|
| Bake condition | Comp. Ex. 1 | Ex. 2 | Ex. 3 | Ex. 4 | Ex. 10 | Comp. Ex. 11 |
| 130 °C/ 17 minutes | 3.4 | 18.2 | 18.4 | 17.6 | 19.7 | 7.3 |

Adhesive viscosity was measured with an Anton Paar Physica MCR 301 rheometer with 25 mm parallel plate and 1 mm gap. Shear condition for the measurement was as follows: Conditioning: Rotation with shear rate at 0.1 s⁻¹ for 60 seconds; Tempering: No shear for 240 seconds; Amplitude test: Oscillation with log increasing strain γ from 0.01 to 10% in 90 seconds (data measured every 3 seconds); Shear phase: Oscillation with 10% strain (γ) at 10 Hz for 120 seconds (data measured every 10 seconds); Re-conditioning: Rotation with shear rate at 0.1 s⁻¹ for 60 seconds; Regenerated mode: Oscillation with 0.05% strain (γ) for 120 seconds (data measured every 10 seconds).

Complex viscosity η^{∗} A1, measured at the condition of y = 0.05% (at 21 seconds), of the above adhesives was measured for its initial value, after 3 days at 43 °C. Samples were conditioned to 35 °C before each measurement. Results of those measurements are shown in Table 3.

**Table 3: Viscosity**

| | Complex viscosity η* A1 | | | | | |
|---|---|---|---|---|---|---|
| Aging Condition | Comp. Ex. 1 | Ex. 2 | Ex. 3 | Ex. 4 | Ex. 10 | Comp. Ex. 11 |
| Initial | 583 | 634 | 634 | 774 | 846 | 584 |
| 3 days at 43 °C | 594 | 829 | 799 | 1270 | 2290 | 732 |

## Claims

1. An adhesive composition comprising:
an epoxy compound; and
a monomeric compound comprising at least one aminimide functional group, wherein the monomeric compound reacts with the epoxy compound upon activation by an external energy source; wherein the monomeric compound is present in an amount from 2-8% by weight based on total weight of the adhesive composition,
wherein the adhesive composition further comprises a reaction product of reactants comprising an amidine and a second component.

2. The adhesive composition of Claim 1, wherein the monomeric compound comprises a reaction product of reactants comprising a monoepoxide, a hydrazine comprising a trivalent nitrogen, and a reactant comprising a carbonyl group.

3. The adhesive composition of any of the preceding Claims, wherein the amidine and the second component form an amidine salt.

4. The adhesive composition of Claim 1 comprising:
an epoxy compound;
an aminimide-containing compound present in an amount of from 2% to 8% by weight based on total weight of the adhesive composition; and
a reaction product of reactants comprising an amidine and a second component,
wherein the epoxy, the aminimide-containing compound, and the reaction product react upon activation by an external energy source.

5. The adhesive composition of Claim 4, wherein the aminimide is a reaction product of a monoepoxide, a hydrazine comprising a trivalent nitrogen, and a reactant comprising a carbonyl group.

6. The adhesive composition of Claim 4 or 5, wherein the second component comprises a phenol.

7. The adhesive composition of any of Claims 4 to 6, wherein the second component comprises carbonic acid, a salt of carbonic acid, carbonate, or combinations thereof.

8. A method for forming a bonded substrate comprising:
applying the adhesive composition of any of the preceding Claims to a first substrate;
contacting a second substrate to the adhesive composition such that the adhesive composition is located between the first substrate and the second substrate; and
curing the adhesive composition.

9. An adhesive comprising the composition of any of the preceding Claims in a cured state.

## Patentansprüche

1. Eine Haftmittelzusammensetzung umfassend:
eine Epoxyverbindung; und
eine monomere Verbindung umfassend mindestens eine funktionelle Aminimidgruppe, wobei die monomere Verbindung mit der Epoxyverbindung bei Aktivierung durch eine externe Energiequelle reagiert; wobei die monomere Verbindung in einer Menge von 2-8 Gew.-%, bezogen auf das Gesamtgewicht der Haftmittelzusammensetzung, vorhanden ist,
wobei die Haftmittelzusammensetzung des Weiteren ein Reaktionsprodukt von Reaktanten umfassend ein Amidin und eine zweite Komponente umfasst.

2. Die Haftmittelzusammensetzung gemäß Anspruch 1, wobei die monomere Verbindung ein Reaktionsprodukt von Reaktanten umfassend ein Monoepoxid, ein Hydrazin umfassend einen dreiwertigen Stickstoff und einen Reaktanten umfassend eine Carbonylgruppe umfasst.

3. Die Haftmittelzusammensetzung gemäß einem der vorhergehenden Ansprüche, wobei das Amidin und die zweite Komponente ein Amidinsalz bilden.

4. Die Haftmittelzusammensetzung gemäß Anspruch 1 umfassend:
eine Epoxyverbindung;
eine Aminimid-haltige Verbindung, die in einer Menge von 2-8 Gew.-%, bezogen auf das Gesamtgewicht der Haftmittelzusammensetzung vorhanden ist; und
ein Reaktionsprodukt von Reaktanten umfassend ein Amidin und eine zweite Komponente,
wobei die Epoxyverbindung, die Aminimid-haltige Verbindung und das Reaktionsprodukt bei Aktivierung durch eine externe Energiequelle reagieren.

5. Die Haftmittelzusammensetzung gemäß Anspruch 4, wobei das Aminimid ein Reaktionsprodukt eines Monoepoxids, eines Hydrazins umfassend einen dreiwertigen Stickstoff und eines Reaktanten umfassend eine Carbonylgruppe ist.

6. Die Haftmittelzusammensetzung gemäß Anspruch 4 oder 5, wobei die zweite Komponente ein Phenol umfasst.

7. Die Haftmittelzusammensetzung gemäß irgendeinem der Ansprüche 4 bis 6, wobei die zweite Komponente Kohlensäure, ein Salz von Kohlensäure, Carbonat oder Kombinationen davon umfasst.

8. Ein Verfahren zur Bildung eines verbundenen Substrats umfassend:
Aufbringen der Haftmittelzusammensetzung gemäß irgendeinem der vorhergehenden Ansprüche auf ein erstes Substrat;
In-Kontakt-Bringen eines zweiten Substrats mit der
Haftmittelzusammensetzung, sodass sich die Haftmittelzusammensetzung zwischen dem ersten Substrat und dem zweiten Substrat befindet; und Härten der Haftmittelzusammensetzung.

9. Eine Haftmittelzusammensetzung umfassend die Zusammensetzung gemäß irgendeinem der vorhergehenden Ansprüche in einem gehärteten Zustand.

## Revendications

1. Composition d'adhésif comprenant :
un composé époxy ; et
un composé monomère comprenant au moins un groupe fonctionnel aminimide, le composé monomère réagissant avec le composé époxy lors d'activation par une source d'énergie externe ; dans laquelle le composé monomère est présent en une quantité de 2 à 8 % en poids par rapport au poids total de la composition d'adhésif,
dans laquelle la composition d'adhésif comprend en outre un produit de réaction de partenaires réactionnels comprenant une amidine et un second composant.

2. Composition d'adhésif selon la revendication 1, dans laquelle le composé monomère comprend un produit de réaction de partenaires réactionnels comprenant un monoépoxyde, une hydrazine comprenant un azote trivalent, et un partenaire réactionnel comprenant un groupe carbonyle.

3. Composition d'adhésif selon l'une quelconque des revendications précédentes, dans laquelle l'amidine et le second composant forment un sel d'amidine.

4. Composition d'adhésif selon la revendication 1, comprenant :
un composé époxy ;
un composé contenant de l'aminimide, présent en une quantité de 2 % à 8 % en poids par rapport au poids total de la composition d'adhésif ; et
un produit de réaction de partenaires réactionnels comprenant une amidine et un second composant,
dans laquelle l'époxy, le composé contenant de l'aminimide, et le produit de réaction réagissent lors d'activation par une source d'énergie externe.

5. Composition d'adhésif selon la revendication 4, dans laquelle l'aminimide est un produit de réaction d'un monoépoxyde, d'une hydrazine comprenant un azote trivalent, et d'un partenaire réactionnel comprenant un groupe carbonyle.

6. Composition d'adhésif selon la revendication 4 ou 5, dans laquelle le second composant comprend un phénol.

7. Composition d'adhésif selon l'une quelconque des revendications 4 à 6, dans laquelle le second composant comprend de l'acide carbonique, un sel d'acide carbonique, un carbonate, ou des associations de ceux-ci.

8. Procédé pour formé un support lié, comprenant les étapes consistant à :
appliquer la composition d'adhésif selon l'une quelconque des revendications précédentes sur un premier support ;
mettre un second support en contact avec la composition d'adhésif de telle façon que la composition d'adhésif soit placée entre le premier support et le second support ; et
faire durcir la composition d'adhésif.

9. Adhésif comprenant la composition selon l'une quelconque des revendications précédentes en un état durci.
